# EUROPEAN PATENT APPLICATION

(11) **EP 3 413 366 A1**
(43) Date of publication of application: **12.12.2018**
(21) Application number: 17747449.1
(22) Date of filing: 01.02.2017
(51) Int. Cl.: H01L 51/44, C07C 317/04

(54) **SOLAR CELL**

(30) Priority: 02.02.2016 JP 2016018107
(71) Applicant: Sekisui Chemical Co., Ltd., Osaka-shi, Osaka 530-0047 (JP)
(72) Inventor: HAYAKAWA, Akinobu, Mishima-gun Osaka 618-0021 (JP); FUKUMOTO, Yuuichirou, Tsukuba-shi Ibaraki 300-4247 (JP); ASANO, Motohiko, Mishima-gun Osaka 618-0021 (JP); YUKAWA, Mayumi, Mishima-gun Osaka 618-0021 (JP); UNO, Tomohito, Mishima-gun Osaka 618-0021 (JP); KUREBAYASHI, Tetsuya, Mishima-gun Osaka 618-0021 (JP)
(74) Representative: Cabinet Beau de Loménie
(86) International application number: PCT/JP2017/003575
(87) International publication number: WO 2017/135293

(57) **Abstract**

The present invention aims to provide a solar cell excellent in photoelectric conversion efficiency, in which reduction in the photoelectric conversion efficiency due to continuous irradiation with light (photodegradation) is suppressed and the photoelectric conversion layer is less likely to suffer corrosion. The present invention provides a solar cell having a structure including: a cathode; an electron transport layer; a photoelectric conversion layer; and an anode stacked in the stated order, the photoelectric conversion layer including an organic-inorganic perovskite compound represented by the formula: R-M-X₃ where R represents an organic molecule, M represents a metal atom, and X represents a halogen or chalcogen atom, the cathode being formed of a titanium material and having an oxide layer on at least one surface.

## Description

### TECHNICAL FIELD

The present invention relates to a solar cell excellent in photoelectric conversion efficiency, in which reduction in the photoelectric conversion efficiency due to continuous irradiation with light (photodegradation) is suppressed and a photoelectric conversion layer is less likely to suffer corrosion.

### BACKGROUND ART

Photoelectric conversion elements equipped with a laminate having an N-type semiconductor layer and a P-type semiconductor layer disposed between opposing electrodes have been conventionally developed. Such photoelectric conversion elements generate photocarriers by photoexcitation so that electrons and holes move through the N-type semiconductor and the P-type semiconductor, respectively, to create an electric field.

Most photoelectric conversion elements currently in practical use are inorganic solar cells which are produced using inorganic semiconductors made of silicon or the like. The inorganic solar cells, however, are utilized only in a limited range because their production is costly and upsizing thereof is difficult. Therefore, organic solar cells produced using organic semiconductors instead of inorganic semiconductors have received attention.

Fullerene is used in most organic solar cells. Fullerene is known to function mainly as an N-type semiconductor. For example, Patent Literature 1 discloses a semiconductor heterojunction film formed using an organic compound serving as a P-type semiconductor, and fullerenes. Fullerene, however, is known to be responsible for degradation of organic solar cells produced using the fullerene (see e.g., Non-Patent Literature 1). Thus, a material that can replace fullerene is desired.

Recently, a photoelectric conversion material having a perovskite structure containing lead, tin, or the like as a central metal, called as an organic inorganic hybrid semiconductor, has been found and proved to have high photoelectric conversion efficiency (see e.g., Non-Patent Literature 2).

### CITATION LIST

### - Patent Literature

Patent Literature 1: JP 2006-344794 A

### - Non-Patent Literature

Non-Patent Literature 1: Reese et al., Adv. Funct. Mater., 20, 3476-3483 (2010)
Non-Patent Literature 2: M. M. Lee et al., Science, 338, 643-647 (2012)

### SUMMARY OF INVENTION

### - Technical Problem

The present inventors studied about the use of an organic-inorganic perovskite compound for a photoelectric conversion layer in a solar cell having a structure including a cathode, an electron transport layer, a photoelectric conversion layer, and an anode stacked in the stated order. The use of an organic-inorganic perovskite compound is expected to improve the photoelectric conversion efficiency of the solar cell.

However, it is newly found out that, though a solar cell having a photoelectric conversion layer containing an organic-inorganic perovskite compound shows high photoelectric conversion efficiency immediately after the start of irradiation with light, the photoelectric conversion efficiency is gradually lowered by continuous irradiation with light (photodegradation). It is also newly found out that the solar cell including a photoelectric conversion layer containing an organic-inorganic perovskite compound may suffer discoloration of the photoelectric conversion layer during the production process of the solar cell or as time advances, possibly resulting in corrosion. The present inventors found that the phenomenon of such corrosion is peculiar to the solar cell including a photoelectric conversion layer containing an organic-inorganic perovskite compound. Such phenomenon has not been reported in relation to other typical solar cells.

The present invention aims to, in consideration of the state of the art, provide a solar cell excellent in photoelectric conversion efficiency, in which reduction in the photoelectric conversion efficiency due to continuous irradiation with light (photodegradation) is suppressed and a photoelectric conversion layer is less likely to suffer corrosion.

### - Solution to problem

The present invention relates to a solar cell having a structure including: a cathode; an electron transport layer; a photoelectric conversion layer; and an anode stacked in the stated order, the photoelectric conversion layer comprising an organic-inorganic perovskite compound represented by the formula: R-M-X₃ where R represents an organic molecule, M represents a metal atom, and X represents a halogen or chalcogen atom, the cathode being formed of a titanium material and having an oxide layer on at least one surface.

The present invention is specifically described in the following.

The present inventors found out that in the case where a cathode that is formed of a titanium material and has an oxide layer on at least one surface is used in a solar cell having a structure including a cathode, an electron transport layer, a photoelectric conversion layer containing an organic-inorganic perovskite compound, and an anode stacked in the stated order, the photoelectric conversion efficiency is further improved, reduction in the photoelectric conversion efficiency due to continuous irradiation with light (photodegradation) is suppressed, and the photoelectric conversion layer is less likely to suffer corrosion. Thus, the present invention was completed.

Though the reason why photodegradation can be suppressed is not exactly clear, the present inventors found out that the photodegradation of the solar cell including a photoelectric conversion layer containing an organic-inorganic perovskite compound is affected by the density of the electron transport layer. In the photoelectric conversion layer containing an organic-inorganic perovskite compound, a reverse electron transfer is particularly likely to occur compared to photoelectric conversion layers containing other semiconductor materials. Presumably, the reverse electron transfer does not occur and the photodegradation is suppressed when the density of the electron transport layer is high, while the reverse electron transfer occurs and the photodegradation is likely to be caused when the density of the electron transport layer is lowered. In particular, when the electron transport layer has a pinhole, the photodegradation instantly occurs. The use of a cathode formed of a titanium material allows formation of an oxide layer (a titanium oxide layer) at the interface between the cathode and the electron transport layer due to natural oxidation, and the titanium oxide layer improves the density of the electron transport layer. In addition, even when the electron transport layer has a pinhole, the titanium oxide layer also serves as an electron transport layer, thereby presumably suppressing photodegradation.

The reason why the photoelectric conversion layer is less likely to suffer corrosion is presumably that the use of a cathode formed of a titanium material suppresses corrosion in the photoelectric conversion layer caused by elemental diffusion from the cathode and an optionally provided substrate. Specifically, though elemental diffusion is likely to occur depending on the type of the cathode and substrate, the use of a cathode formed of a titanium material suppresses elemental diffusion from the cathode. Even when elemental diffusion occurs from the cathode and substrate, an oxide layer (titanium oxide layer) formed at the interface between the cathode and the electron transport layer by natural oxidation presumably suppresses elemental diffusion. The oxide layer formed at the interface between the cathode and the electron transport layer due to natural oxidation preferably includes a gradient oxide layer in which the ratio of titanium atoms to oxygen atoms gradiently increases in the thickness direction toward a portion formed of the titanium material. Such a gradual change in the composition prevents cracking in the oxide layer to presumably further suppress corrosion in the photoelectric conversion layer.

The solar cell of the present invention has a structure including: a cathode; an electron transport layer; a photoelectric conversion layer; and an anode stacked in the stated order.

The term "layer" as used herein means not only a layer having a clear boundary, but also a layer having a concentration gradient in which contained elements are gradually changed. The elemental analysis of the layer can be conducted, for example, by FE-TEM/EDS analysis and measurement of the cross section of the solar cell to confirm the element distribution of a particular element. The term "layer" as used herein means not only a flat thin-film layer, but also a layer capable of forming an intricate structure together with other layer(s).

The cathode is formed of a titanium material. The use of a cathode formed of a titanium material further improves the photoelectric conversion efficiency, suppresses reduction in the photoelectric conversion efficiency due to continuous irradiation with light (photodegradation), and reduces corrosion in the photoelectric conversion layer.

The titanium material may be any material that can generate titanium oxide by oxidation, and examples thereof include titanium metal, mixtures of titanium metal and another metal, titanium alloys such as Ti-6Al-4V, Ti-4.5Al-3V-2Fe-2Mo, and Ti-0.5Pd. Among these, preferred are mixtures of titanium metal and another metal because they are comparatively inexpensive and can lower the resistance value of the cathode to improve the photoelectric conversion efficiency of the resulting solar cell. In the case where a mixture of titanium metal and another metal or a titanium alloy is used as the titanium material, the titanium content is preferably 50% by weight or more from the standpoint of surely achieving the effect of the present invention.

In the case where the titanium material is a mixture of titanium metal and another metal, the titanium material is preferably a laminate including a titanium metal thin film and a thin film of another metal. In such a case, the titanium metal thin film is preferably positioned on the electron transport layer side. The titanium metal thin film may be, for example, titanium foil.

Examples of the metal include aluminum, cobalt, chromium, molybdenum, tungsten, gold, silver, copper, magnesium, and nickel. Among these, preferred are aluminum and cobalt, with aluminum being more preferred, because they are comparatively inexpensive and the resistance value of the cathode is lowered to improve the photoelectric conversion efficiency of the resulting solar cell.

The cathode has an oxide layer on at least one surface. The oxide layer is a layer containing titanium oxide. The oxide layer is preferably positioned on the electron transport layer side of the cathode.

The oxide layer preferably includes a gradient oxide layer in which the ratio of titanium atoms to oxygen atoms gradiently increases in the thickness direction (depth direction) toward a portion formed of the titanium material. Such a gradual change in the composition suppresses cracking in the oxide layer, thereby further suppressing corrosion in the photoelectric conversion layer. The gradient oxide layer may have any thickness. The lower limit of the thickness is preferably 5 nm and the upper limit thereof is preferably 150 nm. The lower limit is more preferably 10 nm and the upper limit is more preferably 100 nm.

The lower limit of the thickness of the oxide layer is preferably 1 nm. When the thickness of the oxide layer is equal to or more than the lower limit, formation of pinholes can be further prevented and reduction in the photoelectric conversion efficiency due to continuous irradiation with light (photodegradation) can be surely suppressed. Moreover, when the thickness of the oxide layer is equal to or more than the lower limit, corrosion in the photoelectric conversion layer due to elemental diffusion from the cathode and an optionally provided substrate can be surely suppressed. The lower limit of the thickness of the oxide layer is more preferably 5 nm, still more preferably 10 nm, particularly preferably 20 nm.

The upper limit of the thickness of the oxide layer is preferably 1,000 nm. When the thickness of the oxide layer is equal to or less than the upper limit, the electrical resistance loss can be reduced to increase the photoelectric conversion efficiency. The upper limit of the thickness of the oxide layer is more preferably 200 nm, still more preferably 100 nm, particularly preferably 50 nm.

For formation of a desired oxide layer, the titanium material is preferably heated. The step of heating the titanium material may be separately provided. Preferably, heating of the titanium material is performed together with another heat treatment in the step of firing the electron transport layer, the step of heat-annealing the photoelectric conversion layer (heat treatment), or the like in the production process of a solar cell. Alternatively, heating in a separate step and heating together with another heat treatment may be both carried out.

The temperature and time for heating the titanium material are not particularly limited. Preferably, heating is performed at 100°C to 500°C for about 1 to 60 minutes.

The formation of the oxide layer can be confirmed by elemental analysis of a cross section of the solar cell by TEM-EDS. The thickness of the oxide layer can be measured by performing X-ray photoelectron spectroscopy (XPS) to evaluate signals of titanium (Ti) and oxygen (O) while conducting Ar etching or C60 etching in the thickness direction (depth direction) of the cathode. Information on the abundance ratio between titanium (Ti) and oxygen (O) in the thickness direction (depth direction) can be obtained by further detailed analysis.

The cathode may be a cathode formed on the surface of a substrate. The substrate is not particularly limited, and may be a rigid-type substrate such as a transparent glass substrate (e.g., a soda-lime glass substrate and an alkali-free glass substrate), a ceramic substrate, and a transparent plastic substrate, or a flexible-type substrate such as a metal thin film formed of a metal other than the titanium material and a plastic thin film. Since the solar cell of the present invention includes a cathode formed of a titanium material, light is preferably incident on the anode side. Thus, the substrate is not necessarily required to have transparency. Accordingly, even a metal thin film formed of a metal other than the titanium material can be used as the substrate without any inconvenience, and the solar cell to be obtained can be used as a flexible solar cell. Moreover, heating treatment during the production process of the solar cell can be performed without any inconvenience.

Alternatively, the solar cell of the present invention may have no substrate. In the case of using a thin film formed of the titanium material as a cathode without a substrate, the solar cell to be obtained can be used as a flexible solar cell. In such a case, the titanium material is preferably a titanium metal thin film. Examples of the titanium metal thin film include titanium foil.

The material of the electron transport layer is not particularly limited, and examples thereof include N-type conductive polymers, N-type low-molecular organic semiconductors, N-type metal oxides, N-type metal sulfides, alkali metal halides, alkali metals, and surfactants. Specific examples thereof include cyano group-containing polyphenylene vinylene, boron-containing polymers, bathocuproine, bathophenanthrene, hydroxyquinolinato aluminum, oxadiazole compounds, benzoimidazole compounds, naphthalene tetracarboxylic acid compounds, perylene derivatives, phosphine oxide compounds, phosphine sulfide compounds, fluoro group-containing phthalocyanine, titanium oxide, zinc oxide, indium oxide, tin oxide, gallium oxide, tin sulfide, indium sulfide, and zinc sulfide. Among these, titanium oxide is particularly preferable because it has excellent affinity with the titanium material constituting the cathode.

The electron transport layer preferably includes a thin-film electron transport layer and a porous electron transport layer stacked on the thin-film electron transport layer. In particular, in the case where the photoelectric conversion layer is a composite film in which an organic semiconductor- or inorganic semiconductor portion is combined with an organic-inorganic perovskite compound portion, the photoelectric conversion layer composite film is preferably formed on the porous electron transport layer because a more complicated composite film (more complicated structure) can be obtained, improving the photoelectric conversion efficiency.

In the present invention, the titanium material is naturally oxidized to form an oxide layer (titanium oxide layer) at the interface between the cathode and the electron transport layer (in particular, the porous electron transport layer), and therefore, a solar cell in which photodegradation is suppressed can be obtained even when the thin-film electron transport layer is not formed. Even in such a case, with an aim of further increasing the density of the electron transport layer, a thin-film electron transport layer may be formed on the oxide layer. In the case where titanium oxide is used as a material of the thin-film electron transport layer, the elemental ratio between titanium and oxygen in the thin-film electron transport layer is preferably 1:2 to 1:1.

The lower limit of the thickness of the thin-film electron transport layer is preferably 5 nm. When the thickness of the thin-film electron transport layer is equal to or more than the lower limit, reduction in the photoelectric conversion efficiency due to continuous irradiation with light (photodegradation) can be further suppressed. The lower limit of the thickness of the thin-film electron transport layer is more preferably 10 nm, still more preferably 20 nm. The upper limit of the thickness of the thin-film electron transport layer is not particularly limited, and is preferably 500 nm. When the thickness of the thin-film electron transport layer is equal to or less than the upper limit, cracking is more likely to be suppressed. The upper limit of the thickness of the thin-film electron transport layer is more preferably 400 nm, still more preferably 300 nm. The thickness of the thin-film electron transport layer as used herein refers to the average distance between the electrode and the porous electron transport layer, and can be measured by observation of a cross section using a transmission electron microscope.

The photoelectric conversion layer contains an organic-inorganic perovskite compound represented by the formula R-M-X₃ wherein R represents an organic molecule, M represents a metal atom, and X represents a halogen or chalcogen atom.

Use of the organic-inorganic perovskite compound in the photoelectric conversion layer can enhance the photoelectric conversion efficiency of the solar cell.

R is an organic molecule and is preferably represented by CₗNₘHₙ (l, m and n each represent a positive integer).

Specific examples of R include methylamine, ethylamine, propylamine, butylamine, pentylamine, hexylamine, dimethylamine, diethylamine, dipropylamine, dibutylamine, dipentylamine, dihexylamine, trimethylamine, triethylamine, tripropylamine, tributylamine, tripentylamine, trihexylamine, ethylmethylamine, methylpropylamine, butylmethylamine, methylpentylamine, hexylmethylamine, ethylpropylamine, ethylbutylamine, imidazole, azole, pyrrole, aziridine, azirine, azetidine, azete, imidazoline, carbazole, methylcarboxyamine, ethylcarboxyamine, propylcarboxyamine, butylcarboxyamine, pentylcarboxyamine, hexylcarboxyamine, formamidinium, guanidine, aniline, pyridine and their ions (e.g., methylammonium (CH₃NH₃)), and phenethylammonium. Among them, methylamine, ethylamine, propylamine, propylcarboxyamine, butylcarboxyamine, pentylcarboxyamine, formamidinium, guanidine and their ions are preferred, and methylamine, ethylamine, pentylcarboxyamine, formamidinium, guanidine and their ions are more preferred. For higher photoelectric conversion efficiency, still more preferred are methylamine, formamidinium and their ions.

M is a metal atom. Examples thereof include lead, tin, zinc, titanium, antimony, bismuth, nickel, iron, cobalt, silver, copper, gallium, germanium, magnesium, calcium, indium, aluminum, manganese, chromium, molybdenum, and europium. Among these, lead or tin is preferred in view of the electron orbital overlap. These metal atoms may be used alone or may be used in combination of two or more thereof.

X is a halogen or chalcogen atom. Examples thereof include chlorine, bromine, iodine, sulfur, and selenium. These halogen or chalcogen atoms may be used alone or may be used in combination of two or more thereof. Among them, a halogen atom is preferred because the organic-inorganic perovskite compound containing halogen in the structure is soluble in an organic solvent and is usable in an inexpensive printing method or the like. In addition, iodine is more preferred because the organic-inorganic perovskite compound has a narrow energy band gap.

The organic-inorganic perovskite compound preferably has a cubic crystal structure where the metal atom M is placed at the body center, the organic molecule R is placed at each vertex, and the halogen or chalcogen atom X is placed at each face center.

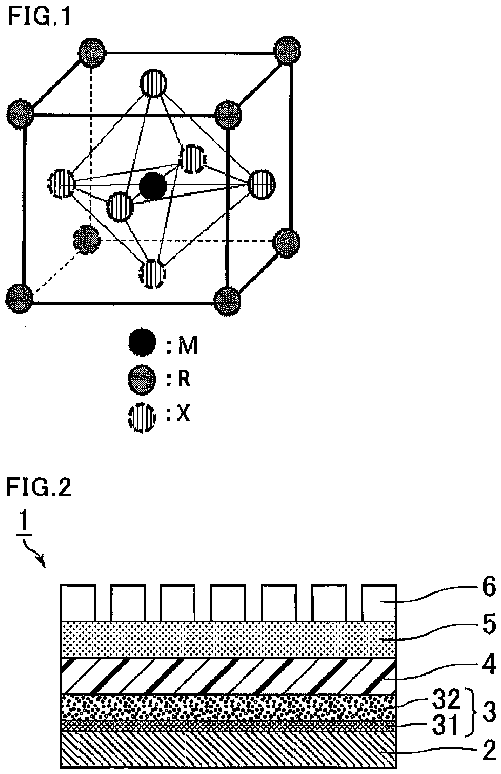

Fig. 1 is a schematic view illustrating an exemplary crystal structure of the organic-inorganic perovskite compound having a cubic crystal structure where the metal atom M is placed at the body center, the organic molecule R is placed at each vertex, and the halogen atom or chalcogen atom X is placed at each face center. Although details are not clear, it is presumed that the direction of an octahedron in the crystal lattice can be easily changed owing to the structure; thus the mobility of electrons in the organic-inorganic perovskite compound is enhanced, improving the photoelectric conversion efficiency of the solar cell.

The organic-inorganic perovskite compound is preferably a crystalline semiconductor. The crystalline semiconductor means a semiconductor whose scattering peak can be detected by the measurement of X-ray scattering intensity distribution.

When the organic-inorganic perovskite compound is a crystalline semiconductor, the mobility of electrons in the organic-inorganic perovskite compound is enhanced, improving the photoelectric conversion efficiency of the solar cell. When the organic-inorganic perovskite compound is a crystalline semiconductor, the reduction in the photoelectric conversion efficiency due to continuous irradiation of the solar cell with light (photodegradation), in particular, photodegradation caused by lowering of the short circuit current is more likely to be suppressed.

The degree of crystallinity can also be evaluated as an index of crystallization. The degree of crystallinity can be determined by separating a crystalline substance-derived scattering peak from an amorphous portion-derived halo, which are detected by X-ray scattering intensity distribution measurement, by fitting, determining their respective intensity integrals, and calculating the ratio of the crystalline portion to the whole.

The lower limit of the degree of crystallinity of the organic-inorganic perovskite compound is preferably 30%. When the degree of crystallinity is 30% or more, the mobility of electrons in the organic-inorganic perovskite compound is enhanced, improving the photoelectric conversion efficiency of the solar cell. When the degree of crystallinity is 30% or more, the reduction in the photoelectric conversion efficiency due to continuous irradiation of the solar cell with light (photodegradation), in particular, photodegradation caused by lowering of the short circuit current is more likely to be suppressed. The lower limit of the degree of crystallinity is more preferably 50%, still more preferably 70%.

Examples of the method for increasing the degree of crystallinity of the organic-inorganic perovskite compound include heat annealing (heat treatment), irradiation with light having strong intensity, such as laser, and plasma irradiation.

The photoelectric conversion layer preferably further contains, in addition to the organic-inorganic perovskite compound, at least one element selected from the group consisting of group 2 elements in the periodic table, group 11 elements in the periodic table, antimony, manganese, neodymium, iridium, titanium, and lanthanum.

The presence of the organic-inorganic perovskite compound and the element in the photoelectric conversion layer suppresses reduction in the photoelectric conversion efficiency due to continuous irradiation of the solar cell with light (photodegradation), in particular, photodegradation caused by lowering of the short circuit current and a fill factor.

Specific examples of the at least one element selected from the group consisting of group 2 elements in the periodic table, group 11 elements in the periodic table, antimony, manganese, neodymium, iridium, titanium, and lanthanum include calcium, strontium, silver, copper, antimony, manganese, neodymium, iridium, titanium, and lanthanum. Among them, preferred are calcium, strontium, silver, copper, neodymium, and iridium. From the standpoint of increasing the initial conversion efficiency, more preferred are calcium, strontium, silver, copper, manganese, and lanthanum, and particularly preferred are calcium, strontium, silver, and copper.

The (molar) ratio of the at least one element selected from the group consisting of group 2 elements in the periodic table, group 11 elements in the periodic table, antimony, manganese, neodymium, iridium, titanium, and lanthanum is not particularly limited. The lower limit of the (molar) ratio of the element to the metal element (M in R-M-X₃) in the organic-inorganic perovskite compound set to 100 is preferably 0.01 and the upper limit thereof is preferably 20. When the (molar) ratio is 0.01 or more, the reduction in the photoelectric conversion efficiency due to continuous irradiation of the solar cell with light (photodegradation), in particular, the photodegradation caused by lowering of the short circuit current density and a fill factor is suppressed. When the (molar) ratio is 20 or less, reduction in the initial conversion efficiency due to the presence of the element can be suppressed. The lower limit of the (molar) ratio is more preferably 0.1 and the upper limit thereof is more preferably 10.

The at least one element selected from the group consisting of group 2 elements in the periodic table, group 11 elements in the periodic table, antimony, manganese, neodymium, iridium, titanium, and lanthanum may be contained in the organic-inorganic perovskite compound by any method. In an exemplary method, a halide of the element is mixed in the solution used for formation of an organic-inorganic perovskite compound layer.

The photoelectric conversion layer may further contain an organic semiconductor or inorganic semiconductor, in addition to the organic-inorganic perovskite compound, as long as the effect of the present invention is not impaired.

Examples of the organic semiconductor include compounds having a thiophene skeleton, such as poly(3-alkylthiophene). Examples thereof also include conductive polymers having a poly-p-phenylenevinylene skeleton, a polyvinylcarbazole skeleton, a polyaniline skeleton, a polyacetylene skeleton or the like. Examples thereof further include: compounds having a phthalocyanine skeleton, a naphthalocyanine skeleton, a pentacene skeleton, a porphyrin skeleton such as a benzoporphyrin skeleton, a spirobifluorene skeleton or the like; and carbon-containing materials such as carbon nanotube, graphene, and fullerene, which may be surface-modified.

Examples of the inorganic semiconductor include titanium oxide, zinc oxide, indium oxide, tin oxide, gallium oxide, tin sulfide, indium sulfide, zinc sulfide, CuSCN, Cu₂O, CuI, MoO₃, V₂O₅, WO₃, MoS₂, MoSe₂, and Cu₂S.

The photoelectric conversion layer containing the organic-inorganic perovskite compound and the organic semiconductor or inorganic semiconductor may be a laminate where a thin-film organic semiconductor or inorganic semiconductor part and a thin-film organic-inorganic perovskite compound part are stacked, or may be a composite film where an organic semiconductor or inorganic semiconductor part and an organic-inorganic perovskite compound part are combined. The laminate is preferred from the viewpoint that the production process is simple. The composite film is preferred from the viewpoint that the charge separation efficiency of the organic semiconductor or inorganic semiconductor can be improved.

The lower limit of the thickness of the thin-film organic-inorganic perovskite compound part is preferably 5 nm and the upper limit thereof is preferably 5,000 nm. When the thickness is 5 nm or more, the thin-film organic-inorganic perovskite compound part can sufficiently absorb light to improve the photoelectric conversion efficiency. When the thickness is 5,000 nm or less, formation of a region in which charge separation cannot be achieved can be suppressed, leading to higher photoelectric conversion efficiency. The lower limit of the thickness is more preferably 10 nm and the upper limit thereof is more preferably 1,000 nm. The lower limit of the thickness is further preferably 20 nm and the upper limit thereof is further preferably 500 nm.

When the photoelectric conversion layer is a composite film where an organic semiconductor or inorganic semiconductor part and an organic-inorganic perovskite compound part are combined, the lower limit of the thickness of the composite film is preferably 30 nm and the upper limit thereof is preferably 3,000 nm. When the thickness is 30 nm or more, light can be sufficiently absorbed, enhancing the photoelectric conversion efficiency. When the thickness is 3,000 nm or less, charge easily arrives at the electrode, enhancing the photoelectric conversion efficiency. The lower limit of the thickness is more preferably 40 nm and the upper limit thereof is more preferably 2,000 nm. The lower limit of the thickness is further preferably 50 nm and the upper limit thereof is further preferably 1,000 nm.

The photoelectric conversion layer may be subjected to heat annealing (heat treatment) after formation of the photoelectric conversion layer. The heat annealing (heat treatment) can sufficiently increase the degree of crystallinity of the organic-inorganic perovskite compound in the photoelectric conversion layer, further suppressing the reduction in the photoelectric conversion efficiency due to continuous irradiation with light (photodegradation).

In the case of performing the heat annealing (heat treatment), the heating temperature of the photoelectric conversion layer is not particularly limited and is preferably 100°C or higher and lower than 200°C. When the heating temperature is 100°C or higher, the degree of crystallinity of the organic-inorganic perovskite compound can be sufficiently increased. When the heating temperature is lower than 200°C, the heat treatment can be carried out without thermally degrading the organic-inorganic perovskite compound. The heating temperature is more preferably 120°C or higher and 170°C or lower. The heating time is not particularly limited and is preferably three minutes or longer and two hours or shorter. When the heating time is three minutes or longer, the degree of crystallinity of the organic-inorganic perovskite compound can be sufficiently increased. When the heating time is two hours or shorter, the heat treatment can be carried out without thermally degrading the organic-inorganic perovskite compound.

These heating operations are preferably performed in vacuum or in an inert gas atmosphere. The dew-point temperature is preferably 10°C or lower, more preferably 7.5°C or lower, still more preferably 5°C or lower.

The anode may be produced from any material, and a conventionally known material may be used. The anode is often a patterned electrode.

Specific examples of the material of the anode include metals such as gold, transparent conductive materials such as CuI, indium tin oxide (ITO), SnO₂, aluminum zinc oxide (AZO), indium zinc oxide (IZO), gallium zinc oxide (GZO), and antimony-doped tin oxide (ATO), and transparent conductive polymers. These materials may be used alone or in combination of two or more thereof.

In the solar cell of the present invention, a hole transport layer may be disposed between the photoelectric conversion layer and the anode.

Examples of the material of the hole transport layer include, but are not particularly limited to, P-type conductive polymers, P-type low-molecular organic semiconductors, P-type metal oxides, P-type metal sulfides, and surfactants. Specific examples thereof include polystyrenesulfonic acid adducts of polyethylenedioxythiophene, carboxyl group-containing polythiophene, phthalocyanine, porphyrin, molybdenum oxide, vanadium oxide, tungsten oxide, nickel oxide, copper oxide, tin oxide, molybdenum sulfide, tungsten sulfide, copper sulfide, tin sulfide, fluoro group-containing phosphonic acid, carbonyl group-containing phosphonic acid, copper compounds such as CuSCN and CuI, and carbon-containing materials such as carbon nanotube and graphene, which may be surface-modified.

The lower limit of the thickness of the hole transport layer is preferably 100 nm. When the thickness of the hole transport layer is equal to or more than the lower limit, reduction in the photoelectric conversion efficiency due to continuous irradiation with light (photodegradation) can be further suppressed. The lower limit of the thickness of the hole transport layer is more preferably 200 nm, still more preferably 300 nm. The upper limit of the thickness of the hole transport layer is not particularly limited, and is preferably 500 nm. When the thickness of the hole transport layer is equal to or less than the upper limit, cracking is more likely to be suppressed. The upper limit of the thickness of the hole transport layer is more preferably 400 nm. The thickness of the hole transport layer as used herein refers to the average distance between the photoelectric conversion layer and the anode, and can be measured by observation of a cross section using a transmission electron microscope.

Fig. 2 is a cross-sectional view schematically illustrating an exemplary solar cell of the present invention.

In a solar cell 1 shown in Fig. 2, an electron transport layer 3 (thin-film electron transport layer 31 and porous electron transport layer 32), a photoelectric conversion layer 4 containing an organic-inorganic perovskite compound, a hole transport layer 5, and an anode 6 are stacked on a cathode 2 in the stated order. Though not illustrated, the cathode 2 has an oxide layer on at least one surface. In the solar cell 1 shown in Fig. 2, the anode 6 is a patterned electrode.

### - Advantageous Effects of Invention

The present invention can provide a solar cell excellent in photoelectric conversion efficiency, in which reduction in the photoelectric conversion efficiency due to continuous irradiation with light (photodegradation) is suppressed and the photoelectric conversion layer is less likely to suffer corrosion.

### BRIEF DESCRIPTION OF DRAWINGS

Fig. 1 is a schematic view illustrating an exemplary crystal structure of an organic-inorganic perovskite compound.
Fig. 2 is a cross-sectional view schematically illustrating an exemplary solar cell of the present invention.

### DESCRIPTION OF EMBODIMENTS

Embodiments of the present invention are more specifically described in the following with reference to, but not limited to, examples.

### (Example 1)

A titanium oxide paste prepared by dispersing titanium oxide nanoparticles (mixture of particles with an average particle size of 10 nm (F6A available from Showa Denko K.K.) and particles with an average particle size of 30 nm (F4A available from Showa Denko K.K.)) in ethanol was applied by spin coating to a surface of a titanium metal thin film (with a naturally oxidized surface) having a thickness of 200 nm as a cathode, followed by irradiation with UV light at 80 mW/cm² for one minute. Thus, a porous electron transport layer was formed.

Separately, lead iodide was reacted with dimethyl sulfoxide (DMSO) in advance to prepare a lead iodide-dimethyl sulfoxide complex. The lead iodide-dimethyl sulfoxide complex was dissolved in N,N-dimethylformamide (DMF) to obtain a 60% by weight coating solution. The resulting coating solution was applied to the electron transport layer by spin coating to a thickness of 200 nm, and an isopropanol solution of methyl ammonium iodide (CH₃NH₃I) adjusted to 8% was further applied thereto by spin coating to react the methyl ammonium iodide with lead iodide, followed by firing at 180°C. Thus, a photoelectric conversion layer containing an organic-inorganic perovskite compound was formed.

Next, a solution was prepared by dissolving, in 25 µL of chlorobenzene, 68 mM of Spiro-OMeTAD (having a spirobifluorene skeleton), 55 mM of t-butylpyridine, and 9 mM of bis(trifluoromethylsulfonyl)imide-lithium salt. The solution was applied by spin coating to form a hole transport layer.

On the obtained hole transport layer was formed an ITO film having a thickness of 100 nm as an anode by electron beam deposition. Thus, a solar cell in which a cathode, an electron transport layer, a photoelectric conversion layer, a hole transport layer, and an anode were stacked was obtained.

### (Examples 2 to 5)

A solar cell was obtained in the same manner as in Example 1, except that the cathode was heated under the firing temperature condition as shown in Table 1 before formation of the porous electron transport layer.

### (Example 6)

A solar cell was obtained in the same manner as in Example 5, except that the thickness of the titanium metal thin film as a cathode was changed as shown in Table 1.

### (Examples 7 and 8)

A solar cell was obtained in the same manner as in Example 1, except that the thickness of the titanium metal thin film as a cathode was changed as shown in Table 1 and the cathode was heated under the firing temperature condition as shown in Table 1 before formation of the porous electron transport layer.

### (Example 9)

A solar cell was obtained in the same manner as in Example 1, except that a thin-film titanium oxide electron transport layer (thickness of 30 nm) was formed by RF sputtering on the surface of the cathode before formation of the porous electron transport layer.

### (Examples 10 and 11)

A solar cell was obtained in the same manner as in Example 1, except that the cathode was heated under the firing temperature condition as shown in Table 1 and a thin-film titanium oxide electron transport layer (thickness of 30 nm) was formed by RF sputtering before formation of the porous electron transport layer.

### (Example 12)

A solar cell was obtained in the same manner as in Example 1, except that a metal thin film as shown in Table 1 was used, instead of the titanium metal thin film as a cathode.

### (Examples 13 and 14)

A solar cell was obtained in the same manner as in Example 12, except that the cathode was heated under the firing temperature condition as shown in Table 1 before formation of the porous electron transport layer.

### (Example 15)

A solar cell was obtained in the same manner as in Example 12, except that the cathode was heated under the firing temperature condition as shown in Table 1 and then a thin-film titanium oxide electron transport layer (thickness of 30 nm) was formed by RF sputtering on the surface of the cathode before formation of the porous electron transport layer.

### (Examples 16 to 19)

A solar cell was obtained in the same manner as in Example 1, except that a metal thin film as shown in Table 1 was used, instead of the titanium metal thin film as a cathode.

### (Comparative Example 1)

A substrate having a titanium metal thin film with a thickness of 200 nm was placed in a glove box in which an oxygen concentration was 10 ppm or less and subjected to hydrofluoric acid treatment in the glove box, whereby an oxidation film on the surface of the titanium metal thin film was removed. Then, a solar cell was obtained in the same manner as in Example 1, except that the operations were performed in the glove box.

### (Comparative Examples 2 to 5)

A solar cell was obtained in the same manner as in Example 1, except that a metal thin film as shown in Table 2 was used, instead of the titanium metal thin film as a cathode.

### (Comparative Example 6)

A solar cell was obtained in the same manner as in Comparative Example 3, except that a thin-film titanium oxide electron transport layer (thickness of 30 nm) was formed by RF sputtering on the surface of the cathode before formation of the porous electron transport layer.

### (Comparative Example 7)

A solar cell was obtained in the same manner as in Example 3, except that the cathode was heated under the firing temperature condition as shown in Table 2 and then a thin-film titanium oxide electron transport layer (thickness of 30 nm) was formed by RF sputtering on the surface of the cathode before formation of the porous electron transport layer.

### (Comparative Examples 8 to 29)

A solar cell was obtained in the same manner as in Example 1, except that a metal thin film as shown in Table 2 was used, instead of the titanium metal thin film as a cathode.

### (Comparative Example 30)

A solar cell was obtained in the same manner as in Example 1, except that a transparent electrode thin film as shown in Table 2 was used, instead of the titanium metal thin film as a cathode and an Au film (thickness of 100 nm) was formed as an anode on the hole transport layer by resistance heating deposition.

### (Comparative Example 31)

A solar cell was obtained in the same manner as in Comparative Example 30, except that a thin-film titanium oxide electron transport layer (thickness of 30 nm) was formed on the surface of the cathode by RF sputtering before formation of the porous electron transport layer.

### (Comparative Examples 32 to 34)

A solar cell was obtained in the same manner as in Example 1, except that a transparent electrode thin film as shown in Table 2 was used, instead of the titanium metal thin film as a cathode and an Au film (thickness of 100 nm) was formed as an anode on the hole transport layer by resistance heating deposition.

### (Comparative Example 35)

A solar cell was obtained in the same manner as in Comparative Example 34, except that a thin-film titanium oxide electron transport layer (thickness of 30 nm) was formed on the surface of the cathode by RF sputtering before formation of the porous electron transport layer.

### <Evaluation>

The solar cells obtained in the examples and comparative examples were evaluated as follows. Tables 1 and 2 show the results.

### (Confirmation of oxide layer and measurement of thickness thereof)

The cathode of each obtained solar cell was subjected to X-ray photoelectron spectroscopy (XPS), while Ar sputtering was performed for etching in the thickness direction (depth direction). The ratio of signals of titanium (Ti) to signals of oxygen (O) was increased and then became constant. The thickness (depth) at which the ratio became constant from the surface of the cathode was measured, and the obtained thickness was taken as the thickness of the oxide layer. Also, the region where the ratio of the signals of titanium (Ti) to the signals of oxygen (O) gradiently increased was measured as the thickness of a gradient oxide layer.

### (Measurement of photoelectric conversion efficiency)

A power source (model 236 available from Keithley Instruments Inc.) was connected between the electrodes of each solar cell. The solar cell was irradiated with light at an intensity of 100 mW/cm² using a solar simulator (Yamashita Denso Corp.) and the photoelectric conversion efficiency was measured. The resulting photoelectric conversion efficiency values were standardized with the photoelectric conversion efficiency of the solar cell obtained in Example 1 set to 1.

### (Photodegradation test)

A power source (model 236 available from Keithley Instruments Inc.) was connected between the electrodes of each solar cell. The solar cell was irradiated with light at an intensity of 100 mW/cm² using a solar simulator (Yamashita Denso Corp.). The photoelectric conversion efficiency was measured right after the start of the irradiation with light and after irradiation with light for one hour. The maintenance rate of the photoelectric conversion performance after the irradiation with light (photoelectric conversion efficiency after irradiation with light for one hour/photoelectric conversion efficiency right after the start of the irradiation with light) was obtained.

### (Evaluation of corrosion in photoelectric conversion layer)

An evaluation sample was prepared by forming a photoelectric conversion layer containing an organic-inorganic perovskite compound on a cathode and firing the photoelectric conversion layer at 180°C in the same manner as in each of the examples and comparative examples. The evaluation sample in which the color of the photoelectric conversion layer was changed from brown (original color of the organic-inorganic perovskite compound) was rated × (Poor). The evaluation sample in which the color of the photoelectric conversion layer was kept brown was rated ○ (Good).

### (Comprehensive evaluation)

When the solar cell had a photoelectric conversion efficiency higher than that of the solar cell obtained in Example 1, kept a maintenance rate of the photoelectric conversion performance of 90% or higher in the photodegradation test, and was rated "○ (Good)" in the evaluation of corrosion in the photoelectric conversion layer, such a solar cell was rated ○ (Good). The solar cell that failed to satisfy any one of these parameters was rated × (Poor).

**[Table 1]**

| | Cathode | | | Titanium oxide-containing oxide layer | | Gradient oxide layer | | Thin-film electron transport layer | | Photoelectric conversion efficiency | Maintenance rate of photoelectric conversion performance , after irradiation with light | | Corrosion in photoelectric conversion layer | Comprehensive evaluation |
|---|---|---|---|---|---|---|---|---|---|---|---|---|---|---|
| | Type | Thickness (nm) | Firing temperature | Presence or absence | Thickness (nm) | Presence or absence | Thickness (nm) | Composition | Thickness (nm) | | (%) | Rating | | |
| Example 1 | Ti | 200 | Not fired | Present | 4 | Present | 4 | Absent | - | 1 | 90 | ○ | ○ | ○ |
| Example 2 | Ti | 200 | 100°c | Present | 6 | Present | 6 | Absent | - | 1.2 | 95 | ○ | ○ | ○ |
| Example 3 | Ti | 200 | 200°c | Present | 11 | Present | 11 | Absent | - | 1.4 | 96 | ○ | ○ | ○ |
| Example 4 | Ti | 200 | 300°c | Present | 35 | Present | 35 | Absent | - | 1.5 | 98 | ○ | ○ | ○ |
| Example 5 | Ti | 200 | 400°c | Present | 80 | Present | 60 | Absent | - | 1.3 | 98 | ○ | ○ | ○ |
| Example 6 | Ti | 500 | 400°c | Present | 80 | Present | 60 | Absent | - | 1.5 | 98 | ○ | ○ | ○ |
| Example 7 | Ti | 500 | 500°c | Present | 200 | Present | 100 | Absent | - | 1.4 | 98 | ○ | ○ | ○ |
| Example 8 | Ti | 500 | 600°c | Present | 280 | Present | 120 | Absent | - | 1.1 | 98 | ○ | ○ | ○ |
| Example 9 | Ti | 200 | Not fired | Present | 33 | Present | 3 | Present | 30 | 1.4 | 90 | ○ | ○ | ○ |
| Example 10 | Ti | 200 | 100°c | Present | 36 | Present | 6 | Present | 30 | 1.5 | 95 | ○ | ○ | ○ |
| Example 11 | Ti | 200 | 200°c | Present | 41 | Present | 11 | Present | 30 | 1.6 | 96 | ○ | ○ | ○ |
| Example 12 | Al/Ti | 200/100 | Not fired | Present | 3 | Present | 3 | Absent | - | 1.4 | 90 | ○ | ○ | ○ |
| Example 13 | Al/Ti | 200/100 | 100°c | Present | 6 | Present | 6 | Absent | - | 1.5 | 94 | ○ | ○ | ○ |
| Example 14 | Al/Ti | 200/100 | 200°c | Present | 11 | Present | 11 | Absent | - | 1.7 | 96 | ○ | ○ | ○ |
| Example 15 | Al/Ti | 200/100 | 200°c | Present | 41 | Present | 11 | Present | 30 | 2 | 97 | ○ | ○ | ○ |
| Example 16 | Co/Ti | 200/100 | Not fired | Present | 3 | Present | 3 | Absent | - | 1.2 | 91 | ○ | ○ | ○ |
| Example 17 | Cr/Ti | 200/100 | Not fired | Present | 3 | Present | 3 | Absent | - | 1.2 | 91 | ○ | ○ | ○ |
| Example 18 | Mo/Ti | 200/100 | Not fired | Present | 3 | Present | 3 | Absent | - | 1 | 93 | ○ | ○ | ○ |
| Example 19 | W/Ti | 200/100 | Not fired | Present | 3 | Present | 3 | Absent | - | 1.1 | 93 | ○ | ○ | ○ |

**[Table 2]**

| | Cathode | | | Titanium oxide-containing oxide layer | | Gradient oxide layer | | Thin-film electron transport layer | | Photoelectric conversion efficiency | Maintenance rate of photoelectric conversion performance after irradiation with light | | Corrosion in photoelectric conversion layer | Comprehensive evaluation |
|---|---|---|---|---|---|---|---|---|---|---|---|---|---|---|
| | Type | Thickness (nm) | Firing temperature | Presence or absence | Thickness (nm) | Presence or absence | Thickness (nm) | Composition | Thickness (nm) | | (%) | Rating | | |
| Comparative Example 1 | Ti | 200 | Not fired | Absent | - | Absent | - | Absent | - | 0 | - | - | ○ | × |
| Comparative Example 2 | Mo | 500 | Not fired | Absent | - | Absent | - | Absent | - | 0 | - | - | ○ | × |
| Comparative Example 3 | Al/Mo | 200/100 | Not fired | Absent | - | Absent | - | Absent | - | 0 | - | - | ○ | × |
| Comparative Example 4 | Co/Mo | 200/100 | Not fired | Absent | - | Absent | - | Absent | - | 0 | - | - | ○ | × |
| Comparative Example 5 | Cr/Mo | 200/100 | Not fired | Absent | - | Absent | - | Absent | - | 0 | - | - | ○ | × |
| Comparative Example 6 | Al/Mo | 200/100 | Not fired | Absent | 30 | Absent | - | Present | 30 | 0.2 | - | - | ○ | × |
| Comparative Example 7 | Al/Mo | 200/100 | 200°C | Absent | 30 | Absent | - | Present | 30 | 0.2 | - | - | ○ | × |
| Comparative Example 8 | Al | 200 | Not fired | Absent | - | Absent | - | Absent | - | 0.2 | - | - | × | × |
| Comparative Example 9 | Cr | 200 | Not fired | Absent | - | Absent | - | Absent | - | 0.1 | - | - | × | × |
| Comparative Example 10 | Co | 200 | Not fired | Absent | - | Absent | - | Absent | - | 0.1 | - | - | × | × |
| Comparative Example 11 | Zn | 200 | Not fired | Absent | - | Absent | - | Absent | - | 0 | - | - | × | × |
| Comparative Example 12 | Al/Zn | 200/100 | Not fired | Absent | - | Absent | - | Absent | - | 0 | - | - | × | × |
| Comparative Example 13 | Co/Zn | 200/100 | Not fired | Absent | - | Absent | - | Absent | - | 0 | - | - | × | × |
| Comparative Example 14 | Cr/Zn | 200/100 | Not fired | Absent | - | Absent | - | Absent | - | 0 | - | - | × | × |
| Comparative Example 15 | Mo/Zn | 200/100 | Not fired | Absent | - | Absent | - | Absent | - | 0 | - | - | × | × |
| Comparative Example 16 | W/Zn | 200/100 | Not fired | Absent | - | Absent | - | Absent | - | 0 | - | - | × | × |
| Comparative Example 17 | Cu | 266 | Not fired | Absent | - | Absent | - | Absent | - | 0 | - | - | × | × |
| Comparative Example 18 | Ni | 200 | Not fired | Absent | - | Absent | - | Absent | - | 0 | - | - | ○ | × |
| Comparative Example 19 | Zr | 200 | Not fired | Absent | - | Absent | - | Absent | - | 0.2 | - | - | ○ | × |
| Comparative Example 20 | Nb | 200 | Not fired | Absent | - | Absent | - | Absent | - | 0.1 | - | - | ○ | × |
| Comparative Example 21 | Ar | 299 | Not fired | Absent | - | Absent | - | Absent | - | 0 | - | - | × | × |
| Comparative Example 22 | In | 200 | Not fired | Absent | - | Absent | - | Absent | - | 0 | - | - | × | × |
| Comparative Example 23 | Sn | 200 | Not fired | Absent | - | Absent | - | Absent | - | 0 | - | - | × | × |
| Comparative Example 24 | Sb | 200 | Not fired | Absent | - | Absent | - | Absent | - | 0 | - | - | × | × |
| Comparative Example 25 | Hf | 200 | Not fired | Absent | - | Absent | - | Absent | - | 0.1 | - | - | ○ | × |
| Comparative Example 26 | Ta | 266 | Not fired | Absent | - | Absent | - | Absent | - | 0.1 | - | - | ○ | × |
| Comparative Example 27 | W | 200 | Not fired | Absent | - | Absent | - | Absent | - | 0.2 | - | - | ○ | × |
| Comparative Example 28 | Pb | 200 | Not fired | Absent | - | Absent | - | Absent | - | 0 | - | - | ○ | × |
| Comparative Example 29 | Bi | 200 | Not fired | Absent | - | Absent | - | Absent | - | 0 | - | - | × | × |
| Comparative Example 30 | ITO | 200 | Not fired | Absent | - | Absent | - | Absent | - | 0.8 | 44 | × | ○ | × |
| Comparative Example 31 | ITO | 200 | Not fired | Absent | 30 | Absent | - | Present | 30 | 1.5 | 18 | × | ○ | × |
| Comparative Example 32 | GZO | 200 | Not fired | Absent | - | Absent | - | Absent | - | 0.5 | 33 | × | ○ | × |
| Comparative Example 33 | SUS | 500 | Not fired | Absent | - | Absent | - | Absent | - | 0 | - | - | × | × |
| Comparative Example 34 | FTO | 500 | Not fired | Absent | - | Absent | - | Absent | - | 0.7 | 66 | × | ○ | × |
| Comparative Example 35 | FTO | 500 | Not fired | Absent | 30 | Absent | - | Present | 30 | 1.8 | 73 | × | ○ | × |

### INDUSTRIAL APPLICABILITY

The present invention can provide a solar cell excellent in photoelectric conversion efficiency, in which reduction in the photoelectric conversion efficiency due to continuous irradiation with light (photodegradation) is suppressed and the photoelectric conversion layer is less likely to suffer corrosion.

### REFERENCE SIGNS LIST

1: Solar cell
2: Cathode
3: Electron transport layer
31: Thin-film electron transport layer
32: Porous electron transport layer
4: Photoelectric conversion layer containing an organic-inorganic perovskite compound
5: Hole transport layer
6: Anode (patterned electrode)

## Claims

1. A solar cell having a structure comprising:
a cathode;
an electron transport layer;
a photoelectric conversion layer; and
an anode stacked in the stated order,
the photoelectric conversion layer comprising an organic-inorganic perovskite compound represented by the formula: R-M-X₃ where R represents an organic molecule, M represents a metal atom, and X represents a halogen or chalcogen atom,
the cathode being formed of a titanium material and having an oxide layer on at least one surface.

2. The solar cell according to claim 1,
wherein the titanium material is titanium metal, a mixture of titanium metal and another metal, or a titanium alloy.

3. The solar cell according to claim 1 or 2,
wherein the oxide layer has a thickness of 1 nm or more and 1,000 nm or less.

4. The solar cell according to claim 1, 2, or 3,
wherein the oxide layer includes a gradient oxide layer in which the ratio of titanium atoms to oxygen atoms gradiently increases in the thickness direction toward a portion formed of the titanium material.

5. The solar cell according to claim 4,
wherein the gradient oxide layer has a thickness of 5 nm or more and 150 nm or less.
